Europäisches Patentamt

European Patent Office

Office européen des brevets

(11) Publication number: **0 116 624**
**B1**

# EUROPEAN PATENT SPECIFICATION

(45) Date of publication of patent specification: **15.04.87**

(51) Int. Cl.⁴: **C 07 C 29/10**

(21) Application number: **83902848.7**

(22) Date of filing: **10.08.83**

(86) International application number:
**PCT/US83/01229**

(87) International publication number:
**WO 84/00748 01.03.84 Gazette 84/06**

(54) PROCESS FOR OLEFIN OXIDE DETOXIFICATION.

(30) Priority: **20.08.82 US 409830**

(43) Date of publication of application:
**29.08.84 Bulletin 84/35**

(45) Publication of the grant of the patent:
**15.04.87 Bulletin 87/16**

(84) Designated Contracting States:
**BE DE FR GB SE**

(56) References cited:
**US-A-2 135 271**
**US-A-2 255 411**
**US-A-2 756 241**
**US-A-3 904 656**
**US-A-4 087 474**
**US-A-4 112 054**
**US-A-4 349 417**

(73) Proprietor: **BUONICORE-CASHMAN
ASSOCIATES, INC.
4695 Main Street
Bridgeport, CT 06606 (US)**

(72) Inventor: **BUONICORE, Anthony, Joseph
685 Church Hill Road
Fairfield, CT 06432 (US)**
Inventor: **DESAI, Pankaj, Ramanlal
124 Shippan Avenue
Stamford, CT 06902 (US)**

(74) Representative: **Goldin, Douglas Michael et al
J.A. KEMP & CO. 14, South Square Gray's Inn
London WC1R 5EU (GB)**

## Description

Technical field

This invention relates to a process for removing olefin oxides, such as ethylene oxide and propylene oxide, from gas compositions containing such, and more particularly to a process for removing ethylene oxide in exhaust gases discharged from ethylene oxide sterilizers.

Ethylene oxide sterilizers are typically utilized, for example, in hospitals and medical product manufacturing facilities for treating articles such as plastic, paper, rubber or the like which cannot withstand heat sterilization. Sterilization is effected when ethylene oxide reacts with contaminating microorganisms to kill or inactivate them.

The sterilization process is typically carried out in a chamber which is evacuated after the articles to be treated are placed within. Either one-hundred percent ethylene oxide or an admixture with an inert diluent such as dichlorodifluoromethane or carbon dioxide is then intro duced to the chamber and allowed to remain therein until the sterilization process is completed, usually from about 1 to 8 hours. After the sterilization period is completed, the gas in the chamber is exhausted and the sterilized articles removed. In order to ensure safe worker exposure levels, it may be necessary to exhaust the chamber in a series of post-evacuations prior to entry into the chamber. Ethylene oxide may be discharged to the environment with each post-evacuation, although concentrations will decrease with each successive evacuation.

A number of evacuations are needed because the product may absorb relatively substantial amounts of the olefin oxide, which off-gasses from the product only gradually. At the end of post-evacuations, the chamber may be ventilated with relatively high flow rates of air. This air containing small amounts of off-gased olefin oxide is ordinarily released into the environment. Even after the sterilized product is removed from the chamber, additional amounts of olefin oxide may be released into the work place because of off-gassing from the product. Because olefin oxide is off-gassed or diffuses out of the product with time, this is the major reason that sterilization plants quarantine the products, preferably in an isolated area after exposure to olefin oxide. The quarantined areas are ordinarily heavily ventilated to reduce worker exposure to the fumes. The small amounts of olefin oxide which are caught-up in the ventilation system are ordinarily released into the environment.

Due to the highly toxic nature of ethylene oxide, special precautions must be taken to assure proper ventilation to the area. Generally, ventilation systems are provided to direct ethylene oxide outside of the building. However, such systems are not entirely satisfactory from an environmental protection standpoint, particularly because there is still the possibility that human receptors outside of the building may unknowingly come into contact with the ethylene oxide before it has been sufficiently dispersed in the ambient atmosphere. Moreover, unfavorable meterological conditions can contribute to unacceptably high ground level ambient ethylene oxide concentrations and/or hazardous in-plant worker exposure levels due to ethylene oxide recirculation back into the building. Such concern becomes paramount with the acceptable ethylene oxide worker exposure level reduced below one part per million. Consequently, a means must be provided to control the venting of ethylene oxide, particularly in any areas where human receptors can be exposed to such releases.

In view of increased concern over the environment, various methods have been devised by the art in an attempt to remove toxic olefin oxides, such as ethylene oxide and propylene oxide, from exhaust gases containing such. For example, U.S. Patent No. 3,851,043 to Gunther discloses a method of treating exhaust gases from ethylene oxide sterilizers by passing the exhaust gas through a column containing a strong acid, cationic active ion exchange resin in the hydrogen form. The Gunther patent also discloses the use of an oxidizing resin manganese greensand in place of the ion exchange resin. The process taught by Gunther is a single step, batch process, having the disadvantage that the absorption beds must be either regenerated or disposed of once they have become saturated.

It is well known in the art that olefin oxides can be hydrolized to alkylene glycols, which in dilute solutions are not considered to be environmentally hazardous. These acid catalyzed reactions are described by Morrison and Boyd, *Organic Chemistry,* Second Edition, pp. 887ff, 1966, and U.S. Patent Nos. 1,641,710, 2,636,906 and 4,165,440.

U.S. Patent No. 4,112,054 to Feingold et al discloses a method of removing ethylene oxide from gaseous mixtures by passing the gas through an aqueous acidic solution to simultaneously dissolve the ethylene oxide and convert the ethylene oxide to ethylene glycol and other products at a pH of less than 4 and an initial temperature of 50°C. The Feingold et al patent is directed toward relatively small hospital sterilizers, and particularly toward a sterilizing means which can be rendered portable, to afford quick and efficient use. In addition, the Feingold et al process is disclosed as having an efficiency of 97% using acids such as oxalic acid, sodium bisulfate, hydrochloric acid, and hydrobromic acid. Of the acids described by Feingold et al as being most efficient, halogenated acids will produce halohydrin by-products, i.e., ethylene bromo- and chlorohydrins, which are considered highly toxic substances, and oxalic acid is extremely expensive and not cost effective. In addition, in their process, Feingold et al absorb and convert the olefin in one step. Therefore, the absorption of ethylene oxide cannot be optimized, and the conversion to alkylene glycols likewise cannot be optimized since the exothermic heat of solution using an acidic solution to absorb the olefin oxide reduces the dissolution rate. The Feingold et al patent is also a batch process and limited in application to sterilizers which do not use steam ejectors for evacuation.

U.S. Patent No. 2,135,271 to Balcar is directed toward a method of optimizing glycol production. Disclosed is a cyclic method wherein an olefin oxide is absorbed and converted via thermal hydrolysis to an alkylene glycol, and the alkylene glycol solution is then recycled back to the absorber to absorb more olefin oxide until the concentration of the alkylene glycol reaches a point where it is commercially economical to separate the glycol from solution. The Balcar patent is not concerned with the efficiency of removing olefin oxides from gaseous mixtures.

Accordingly, it is an object of this invention to provide a highly efficient process for removing olefin oxides from gas mixtures containing olefin oxides in order that environmental hazards may be greatly reduced.

A further object of the present invention is to provide for the removal of olefin oxides from gas compositions in a process useable on an industrial scale.

A further object of this invention is to remove at ambient pressure more than about 99% of olefin oxides from a gas composition containing such.

A further object of the invention is to provide a process for converting more than about 99% of the olefin oxides into substances which are not environmentally dangerous.

A further object of the invention is to provide a process wherein olefin oxides can be efficiently converted to alkylene glycols using sulfuric acid as a catalyst, which does not convert to toxic substances, and is relatively inexpensive to use.

A further object of the invention is to provide a process wherein olefin oxides that are "off-gassed" from the product either during chamber ventilation or in quarantine areas and released into a ventilation system can be removed from the ventilation streams.

Further objects of the invention will become apparent from the following description and claims.

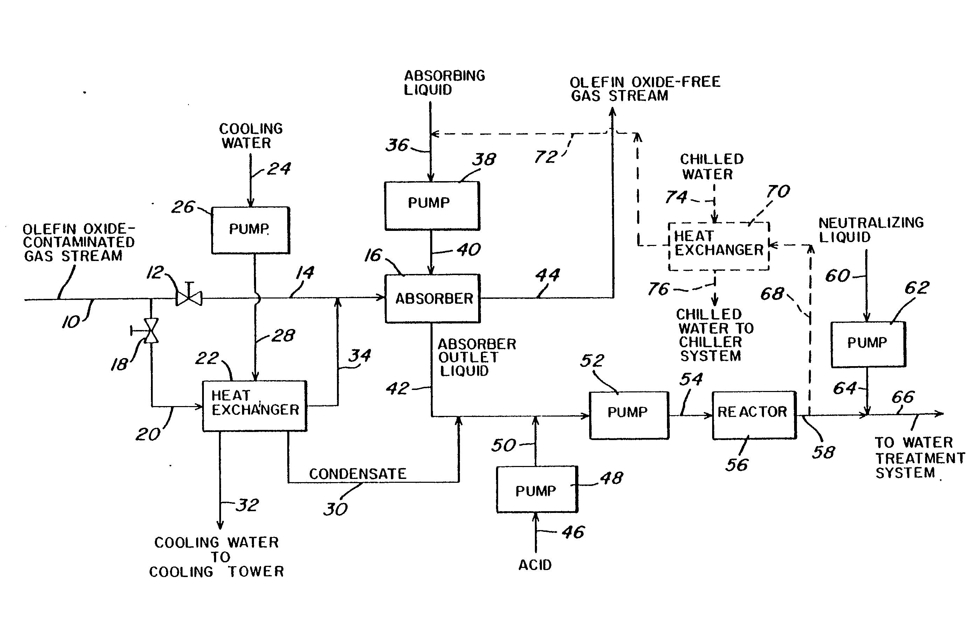

The figure illustrates a flow diagram for the claimed olefin oxide detoxification process.

This invention relates to a method for removing certain olefin oxides from gas compositions, and converting such into alkylene glycols. The process is essentially a two-stage process wherein the olefin oxide is absorbed in a neutral or acidic absorbing liquid and is thereafter transferred to a reactor, together with an acid catalyst, wherein the absorbed olefin oxide is converted to alkylene glycols. Thereafter, the reaction products, consisting essentially of water, acid catalyst, and alkylene glycol, from the reactor can be recycled back to the absorber, preferably after appropriate cooling, or neutralized using an alkali, and the neutralized, non-toxic reaction product stream can be either transferred to a water treatment system, or safely discharged to a drain. Accordingly, the present invention provides a process for removing at substantially ambient pressure an olefin oxide having 2 or 3 carbon atoms from a gas composition containing said olefin oxide and converting the olefin oxide to products including alkylene glycol which process comprises:

contacting said gas composition with an adsorbent capable of adsorbing an olefin oxide;

regenerating the olefin oxid from the adsorbent periodically to provide a more concentrated olefin oxide gas composition;

contacting said concentrated olefin oxide gas composition with an aqueous absorbing liquid at substantially ambient pressure and an initial temperature less than about 80°F (27°C), causing absorption of at least about 97% of the olefin oxide into the aqueous liquid;

feeding the aqueous liquid containing the absorbed olefin oxide at a temperature of less than about 80°F (27°C) and an acid catalyst into a reactor, said reactor being at atmospheric pressure or slightly above atmospheric pressure;

reacting said aqueous liquid for a time sufficient to convert at least 97% of the olefin oxides to products including an alkylene glycol while continuously removing sufficient quantities of aqueous liquid from the reactor to keep the volume of solution in the reactor substantially constant; and

recycling the aqueous liquid leaving the reactor, back to the absorber for further absorption of olefin oxide. The aqueous reaction product stream may be neutralized with an alkali.

Further, the present invention provides a means for removing an olefin oxide from plant ventilation streams including sterilization chamber ventilation following post-evacuations. The "off-gassed" olefin oxide from the ventilation stream is captured on an adsorbent such as a carbon bed. Periodically, when the apparatus for evacuating the sterilizer is not ordinarily in use, the carbon in the carbon bed is regenerated by removing the olefin oxide therefrom by vacuum and subjecting the removed olefin oxide to the process described above for the treatment of the olefin oxide that is removed from the sterilizer.

The process flow diagram of the olefin oxide detoxification system is shown in the figure. The olefin oxide contaminated gas stream enters the system through line 10. The olefin oxide, such as ethylene oxide, concentration can range anywhere from 0 to 100% by volume. In view of the explosive nature of ethylene oxide in air, rarely will a 100% ethylene oxide discharge stream to the atmosphere be encountered in industrial practice. More typically, the gas stream will consist of an ethylene oxide admixture with an inert diluent such as dichlorodifluoromethane carbon dioxide or steam. Gas streams with a non-condensable diluent, such as dichlorodifluoromethane or carbon dioxide, are directed through valve 12 into line 14 to absorber 16. Gas streams with a condensable diluent, such as steam discharged from an ejector evacuating an ethylene oxide sterilizer, are directed through valve 18 into line 20 to heat exchanger 22. When valve 12 is open, valve 18 is closed. When valve 18 is open, valve 12 is closed. Cooling water supplied via line 24 is pumped to heat exchanger 22 by pump 26 via line 28 to condense the condensable portion of the ethylene oxide contaminated gas stream. Liquid condensate containing a portion of the ethylene oxide is then fed via line 30 and mixed with the absorber 16 outlet fluid stream. Cooling water from heat exchanger 22 is fed to a cooling tower or the like via line 32. Vapors from heat exchanger 22 are directed through line 34 to absorber 16. The gas stream is contacted with an aqueous liquid in absorber 16 wherein the olefin oxide present in the gas stream is absorbed by the aqueous liquid and removed from the gas stream.

Absorber 16 is an absorption vessel, typically either filled with a bed of packing materials or containing any of the various types of trays (e.g., sieve trays, bubble-cap trays, etc.) arranged one above the other, which provides a large surface area for contact between the aqueous absorbing liquid and the olefin oxide-contaminated gas. The transfer of olefin oxide from the gas phase to the liquid phase takes place primarily on the packing surface or on the trays. Any type of absorber which provides sufficient contact surface area may be used for olefin oxide absorption. The absorbing liquid which may be the reactor product stream after appropriate cooling is supplied to the detoxification system via line 36 and is pumped to the absorber by pump 38 via line 40. The absorbing liquid absorbs olefin oxide from the gas stream in the absorber and leaves the absorber via line 42. Other ethylene oxide contaminated liquid streams such as the condensate from heat exchanger 22 or the discharge water from any liquid ring vacuum pumps on the sterilizers may be added to the absorber outlet liquid stream in line 42. The substantially olefin oxide-free gas leaves the absorber via line 44 and may be discharged into the atmosphere. Under appropriate operating conditions, at least about 99% of the olefin oxide in the gas composition is absorbed.

An acid is added to the olefin oxide liquid solution upstream of reactor 56 to achieve the desired pH. The acid is supplied to the detoxification system via line 46 and is added to the liquid by metering pump 48 via line 50. The now acidulated liquid is pumped to reactor 56 by pump 52 via line 54. Olefin oxide reacts with water to form alkylene glycol in the reactor 56. The extent of olefin oxide conversion to alkylene glycol depends primarily upon the liquid pH, reaction temperature and residence time. Sufficient residence time and adequate mixing are provided in reactor 56 to convert, under appropriate operating conditions, at least about 99% of the olefin oxide to alkylene glycol under the prevailing conditions of temperature and pH. The reaction products leave the reactor via line 58 and essentially consist of water, acid catalyst and alkylene glycol. The acid in the aqueous solution leaving the reactor can be neutralized with an alkali. The neutralizing agent is supplied to the detoxification system via line 60 and is added to the liquid stream by pump 62 via line 64. The neutralized liquid stream leaves the detoxification system via line 66 and may be discharged directly to a sanitary drain or directed to a water treatment facility. Alternatively, in a recycle operation, the aqueous solution of ethylene glycol leaving the reactor is sent to heat exchanger 70 via line 68, where it is cooled using chilled water. The cold stream of ethylene glycol solution leaving heat exchanger 70 is recycled back to the absorbing liquid supply line 36 via line 72, and pumped back to the absorber by pump 38 via line 40 for further absorption of olefin oxide. The chilled water is supplied to heat exchanger 70 via line 74 and is sent back to a chiller system for reuse via line 76. In the recycle process, acid need be introduced only at the beginning of the operation since the acid catalyst is not consumed in the reaction.

The process of the present invention is designed to remove olefin oxides, having either 2 or 3 carbon atoms, i.e., ethylene oxide or propylene oxide, from a gas stream, and to chemically convert the oxides by hydrolysis to less environmentally hazardous products.

The absorption of the olefin oxide is carried out in any conventional type of absorber, for example, packed towers, plate columns, etc., which provide a high degree of gas-liquid contact. Any conventional type of packing material may be used. Preferably, the absorption is carried out in a packed counter-current scrubber. Preferably, the scrubber will contain a packing material which provides a large contact surface area per unit packed volume. For example, plastic packing material having a diameter of about 1/2" to 2" (1.27 to 5.08 cm) may be used.

For the purposes of the invention, any aqueous liquid that satisfactorily absorbs olefin oxides may be used. For example, waste water that is recycled from other processes may be used. In general, the preferred pH range of the absorbing liquid is between 0.2 and 8, more preferably between about 0.5 and 7.5. For non-recycle operation, the preferred pH range of the absorbing liquid is between 4 and 8, more preferably between about 6.5 and 7.5. In a recycle process, however, the preferred pH range of the absorbing liquid is between 0.2 and 4, more preferably between about 0.5 and 2. The amount of ethylene glycol in the absorbing liquid ranges preferably from 0 to 70% by weight, more preferably from 0 to 40% by weight. The absorbing liquids having pH values outside the preferred ranges may also be used as pH values will have a minimum impact on the absorption process and will primarily affect acid catalyst requirements. If the pH of the absorbing liquid is low, the relatively small residence time of the liquid in the absorption column as compared to that in the reactor is insufficient to achieve any significant degree of conversion in the absorber. However, less acid catalyst will be required. On the other hand, if the pH of the absorbing liquid is high, acid catalyst requirements upstream of the reactor will be increased in order to achieve the desired degree of conversion to glycol in the reactor.

The absorption of olefin oxide is favored by high liquid-to-gas ratios. The process is normally conducted at liquid-to-gas ratios, i.e., absorbing liquid flow rate/gas stream flow rate ratios of about 40 to 100 gallons/1000 ft$^3$ (5.35 to 13.38 litres/m$^3$). Preferably, the liquid-to-gas ratios will be maintained at about 60 to 70 gallons/1000 ft$^3$ (8.02 to 9.36 litres/m$^3$). The absorbing liquid may be supplied to the absorber through spray nozzles installed above the main packed volume or trays. Gas containing the olefin oxide is introduced at the bottom. The liquid and the gas streams are made to contact counter-current in the absorber where more than about 97% to 98%, and preferably more than about 99% of the olefin oxide is absorbed from the gas stream into the absorbing liquid with the absorption column operating essentially at ambient pressure. The clean gas exits the absorber at the top. The liquid leaving the bottom of the absorber is an aqueous solution of olefin oxide and/or alkylene glycol.

The amount of olefin oxide in the gas stream can be between 0 and 100% by volume. More typically, the olefin oxide in the gas stream will range from about 1000 ppm to 50% by volume. Most typically, the amount of olefin oxide in the gas stream will be from about 1 to 30% by volume.

Generally, the gas temperature at the inlet of the absorber will be less than about 500°F (260°C). It is preferred that the temperature at the gas inlet be less than about 230°F (110°C), and generally the temperature will be less than about 140°F (60°C). In general, the gas may flow into the absorber at any rate that may be convenient. In the case of an extremely high gas flow rate, the process can merely ba adjusted by increasing the flow rate of the absorbing liquid so long as the absorber has been designed to treat the extremely high gas flow rate.

For the purposes of this invention, the liquid solution upon leaving the absorber will typically contain less than about 5% by weight of olefin oxide, and 0 to 40% by weight of alkylene glycol. In general, the absorbing liquid entering the absorber contains less than about 100 ppm by weight of an olefin oxide and less than about 40% by weight of alkylene glycol.

The absorbing liquid entering the absorber can be maintained at less than about 80°F (27°C). Preferably, the temperature will be less than about 70°F (21°C) and, most preferably, the temperature will be less than about 60°F (16°C). If tap water or fresh water is used, the temperature of the absorbing liquid will vary as the temperature of the water in the pipes, and may be as low as about 35°F (2°C). As will be appreciated by those skilled in the art, the absorbing liquid will lower the temperature of the gas in the gas stream, and in the case where the gas stream temperature is extremely high, lower absorbing liquid temperatures will be preferred since absorption is facilitated at lower temperatures.

For convenience, in the preferred embodiment of the present invention, the acid catalyst is added to the liquid stream after it leaves the absorber and before it reaches the reactor. Any acid producing hydrogen ions in solution can be used. Mineral acids, such as $H_3PO_4$, HCl, HBr, HF, and $H_2SO_4$, are preferred. Most preferred is sulfuric acid. As is known to those skilled in the art, dilution of concentrated acids, and particularly dilution of sulfuric acid, is strongly exothermic. Accordingly, the temperature of the absorbing liquid entering the reactor may be considerably higher than the temperature of the absorbing liquid leaving the absorber.

After addition of the catalyst, the absorbing liquid solution is processed in a reactor operating in a continuous mode. Any type of reactor can be used in the present process that provides the proper degree of mixing and residence time for the chemical reaction to occur. For example, a constant flow stirred tank reactor or a plug flow type reactor can be used. The olefin oxide solution is fed into the reactor at a constant rate and the processed liquid is discharged at the same rate. In general, any liquid flow rate can be handled by the reactor as long as the proper degree of mixing and residence time, for the size of the reactor, is maintained.

Hydrolysis of the olefin oxide to the corresponding alkylene glycol is favored by low pH conditions. As discussed above, in the preferred embodiment of the invention, in general, the absorbing liquid entering the reactor will contain less than about 5% by weight of olefin oxide, and less than about 40% by weight of alkylene glycol. The proper degree of mixing is provided in the reactor. The retention time needed for substantially complete hydrolysis of the olefin oxide is dependent on the hydrogen ion concentration in the reactor, the temperature and the liquid flow pattern in the reactor. In general, for a tubular flow reactor treating an ethylene oxide contaminated liquid stream at a pH between about 0.5 to 2.0 and a temperature between about 70°F to 100°F (21°C to 38°C), the retention time of the liquid in the reactor can range between about 1 minute and 3 hours. The retention time will range preferably between about 1 minute and 1 hour, and most preferably between about 5 minutes and 20 minutes.

The temperature of the liquid entering the reactor is less than about 80°F (27°C). Since, at higher temperatures, the olefin oxides and other gases are less soluble and will escape from the liquid, the pressure in the reactor may be slightly above atmospheric. In general, chemical reaction will take place in the reactor at atmospheric pressure or slightly above. Under the operating conditions of the process, more than about 99% of the olefin oxide present in the liquid stream can react with water to form alkylene glycol.

The conversion of the olefin oxide to alkylene glycol can be facilitated using any acid which ionizes to provide hydrogen ions in solution, for example, sulfuric acid, hydrochloric acid, phosphoric acid, etc. As discussed above, for reasons of economics, and safety, the most preferred catalyst is sulfuric acid. In general, the concentration of hydrogen ion catalyst used in the process will be about 0.1 molar, although the preferred range is from about .01 to 1 molar. The most preferred range of hydrogen ion concentration is from 0.03 to 0.3 molar since the cost of the catalyst is reduced, and because neutralization can be more easily achieved when lower concentrations of acid are used.

In general, the mole ratio of acid to olefin oxide will be between about .001 to 250, preferably between about .005 and 200, and most preferably between about .01 to 25, and the pH of the reactants entering the reactor will be preferably about 0 to 2.

The liquid stream leaving the reactor in the process of the invention is an aqueous solution of alkylene glycol which contains a negligible amount of unreacted olefin oxide. The concentration of alkylene glycol in the liquid leaving the reactor typically will be less than about 70% by weight. Generally, for the purposes of the preferred embodiment, it will be less than about 40% by weight. This liquid stream is either recycled after appropriate cooling back to the absorber or neutralized with an alkali and safely disposed of.

For purposes of this invention, for once-through operation, after the liquid stream leaving the reactor has been neutralized, it will be released into the environment. Any alkaline chemical which can produce hydroxyl ions in solution can be used to neutralize the hydrogen ions of the catalyst. For example, alkali metal and alkaline earth metal hydroxides, such as potassium hydroxide, magnesium hydroxide, calcium hydroxide, or sodium hydroxide, etc. can be used. Most preferably, sodium hydroxide will be used. In recycle operation, the concentration of alkylene glycol in the aqueous solution leaving the reactor will increase this time as more and more olefin oxide is absorbed in the absorber and hydrolyzed to alkylene glycol in the reactor. When the alkylene glycol concentration in the reactor outlet stream reaches at least about 40% by weight, the relatively concentrated, aqueous solution of alkylene glycol will be removed from the entire system and disposed of safely. The system will subsequently be filled with fresh water for olefin oxide absorption and hydrolysis.

The alkylene oxide hydrolysis reaction takes a finite amount of time and, even under optimum conditions, there will always be, at any given time, unconverted olefin oxide in solution. This unconverted olefin oxide in solution is very sensitive to temperature, and a relatively small rise in temperature can cause a decrease in the olefin oxide solubility in the absorbing liquid composition. Since dilution of the preferred sulfuric acid catalyst is a strongly exothermic reaction, and hydrolysis of the olefin oxide is also an exothermic reaction, the temperature of the liquid in the reactor, in general, will be higher than the temperature of the liquid leaving the absorber.

Since the reactor is closed, partial pressures of the gases dissolved in the liquid from the scrubber may raise the pressure in the reactor to slightly above atmospheric. Since the reactor is closed, however, as olefin oxide is hydrolyzed to the corresponding alkylene glycol, gas will be absorbed, and consequently also hydrolyzed, thereby making possible complete hydrolysis of nearly all of the olefin oxide absorbed from the gas stream. Complete hydrolysis takes place, despite the fact that there is a slight lag or induction period before hydrolysis of an olefin oxide begins when using sulfuric acid as a catalyst. This lag or induction period is presumed to be one of the reasons that prior art processes were not capable of removing more than 94% of the olefin oxide when using a single step absorption-reaction process with a sulfuric acid catalyst.

On an industrial scale, more than one absorber may be used. In such a case, olefin oxides which have been absorbed by the absorbing liquid in more than one absorber may be fed into a single reactor. In addition, other olefin oxide liquid solutions from other contamination sources may also be fed into the reactor and similarly may be converted to the corresponding alkylene glycol.

Also, some gas streams may also contain steam (for example, the exhaust gas from a sterilizer equipped with steam ejectors for evacuation), and the gas temperature can be as high as about 300°F (149°C) or more. If the olefin oxide-contaminated gas mixture contains steam, the gas may be passed through a heat exchanger as illustrated in the figure. The heat exchanger can be used to condense virtually all of the steam present in the contaminated gas. Cooling water can be used for this purpose. The heat exchanger provides an indirect contact between the cooling water and the gas stream to reduce the gas temperature below saturation temperature. This condenses most of the steam and a small portion of olefin oxide present in the gas stream, and simultaneously causes a rise in the cooling water temperature. The relatively hot cooling water leaves the heat exchanger and is sent to a cooling tower for reuse. The relatively cool gas leaves the heat exchanger and enters the absorber. The condensate primarily would consist of a very dilute solution of olefin oxide in water. This condensate stream can be mixed with the liquid stream from the absorber for further treatment in the reactor. The heat exchanger may also be used to cool the olefin oxide-contaminated gas stream, if necessary, even if the gas stream does not contain steam. The heat exchanger may be excluded from the detoxification system if there are no cooling requirements.

As described above, the product, after being subjected to sterilization, is aerated in the chamber for a certain period of time and/or isolated in an isolation area since it retains a certain amount of olefin oxide thereon, which cannot be removed during the evacuation of the sterilizer. The off-gased olefin oxide is diluted in the ventilation air and removed by ventilating the chamber and/or isolation area. In order to prevent as much olefin oxide as possible from escaping into the environment, it is preferred that additional steps be taken to remove olefin oxide from the ventilation streams of the isolation area and the chamber.

Since the above described apparatus is very cost sensitive to the flow rate of gas which is to be treated, it is not economically practical to design the apparatus large enough to handle the high gas flow rate ventilation streams encountered while the product is off-gassing. Therefore, the apparatus is sized to operate only during the evacuation(s) of the sterilizer.

Since the ventilation gas flow rates are relatively large compared to the vacuum pump flow rates during evacuation of the sterilization chamber, and the ventilation streams contain relatively low concentrations of olefin oxide, and since the ventilation systems (except chamber ventilation) operate continuously, we have found that it is most practical to adsorb and concentrate the "off-gassed" olefin oxide as it is removed from the ventilation stream onto an adsorbent, for example, a carbon adsorbent, and to regenerate the adsorbent on, for example, a daily basis, and subject the olefin oxide removed from the adsorbent to the above described process.

A carbon adsorbent is preferably used, although it should be recognized that any adsorbent suitable for adsorbing an olefin oxide can be used. Using such a method, the apparatus described above may be used to neutralize the olefin oxide at a time when the apparatus would not ordinarily be operating.

Air, from the ventilation system, containing olefin oxide, which is removed from the "off-gassing" product, is passed through air ducts over a carbon bed. Preferably, activated carbon, such as activated charcoal, is used. Since the olefin oxide concentrations in the ventilation system are extremely low, it ordinarily will take a considerable amount of time to saturate a carbon bed. Therefore, the system could be designed for regeneration once every 24 hours. Longer or shorter times may be used depending on the needs. In this manner, regeneration can take place at a time when the vacuum pump and the apparatus are not ordinarily in use. The carbon beds used in the ventilation system can be regenerated individually, and it is therefore possible to develop a sequence for regenerating individual carbon beds while the carbon beds associated with separate vent systems continue to adsorb olefin oxide from the ventilation streams. Regeneration of each carbon bed can be cycled appropriately and controlled automatically by the apparatus.

It is noted that the size of the carbon bed will control the frequency of the regeneration needed. The size of the carbon bed and the frequency of regeneration can be determined by site-specific plant requirements and operating conditions. In the preferred embodiment of the invention, just prior to regeneration of the carbon bed, the individual carbon bed can be isolated by an appropriate valving system. During regeneration, the vacuum damper would open and the existing sterilizer vacuum pump, described above, would pull off the adsorbed olefin oxide. This concentrated olefin oxide stream would then be discharged into the apparatus as described above. As is recognized by those skilled in the art, the carbon bed system is designed to structurally withstand the required vacuum conditions. Also, appropriate precautions are taken to prevent explosion hazards. By the above described means, more effective use of the existing apparatus can be made, and more total ethylene oxide can be detoxified.

The following examples are provided to illustrate the preferred embodiments of the invention and are not intended as limitations on the process.

Example 1

187 actual cubic feet per minute (5295 litres per minute) of a gas mixture at 60°F (16°C) consisting of 24.2% by weight ethylene oxide and 75.8% by weight nitrogen was discharged to a 12 inch (30.5 cm) diameter column packed to a height of 6.0 feet (1.83 m) with 1 inch (2.54 cm) TELLERETTE packing manufactured by the Ceilcote Company in Berea, Ohio. 12.3 gallons per minute (46.6 litres per minute) of fresh water at 60°F (16°C) was used as the scrubbing medium to absorb ethylene oxide using counter-current contact. 99.25% of the ethylene oxide was removed from the gas stream entering the absorber. The absorbing liquor exiting the bottom of the packed tower at 60°F (16°C) containing 3.6% by weight ethylene oxide was pumped to a holding tank from which the ethylene oxide contaminated solution was pumped at a controlled flow rate of 2 gallons per minute (7.57 litres per minute) to a 175 gallon (662 litre) continuous stirred tank reactor containing internal baffles and a 1/2 horsepower (373 W) agitator. 5.0 molar sulfuric acid solution was fed to the reactor at a flow rate of 150 milliliters per minute to maintain a pH of 1.01 in the reactor. The reactor temperature was 83°F (28°C) and liquid residence time 87.5 minutes. 98.0% of the ethylene oxide in solution was converted to ethylene glycol. All ethylene oxide and ethylene glycol determinations were made by gas chromatography with simultaneous measurements at the inlet and outlet of the absorber and reactor.

Example 2

202 actual cubic feet per minute (5720 litres per minute) of a gas mixture at 60°F (16°C) consisting of 31.8% by weight ethylene oxide and 68.2% by weight nitrogen gas was discharged to a 12 inch (30.5 cm) diameter column packed to a height of 6.0 feet (1.83 m) with 1 inch (2.54 cm) TELLERETTE packing. 12.4 gallons per minute (46.9 litres per minute) of fresh water at 60°F (16°C) was used as the scrubbing medium to absorb ethylene oxide using counter-current contact. 99.34% of the ethylene oxide was removed from the gas stream entering the absorber. All ethylene oxide determinations were made by gas chromatography with simultaneous measurements at the inlet and outlet of the absorber.

Example 3

To evaluate the importance of pH on the ethylene oxide-ethylene glycol reaction, the pH in the reactor was increased to 2.10. An aqueous liquor at 60°F (16°C) containing 3.4% by weight ethylene oxide was pumped at a controlled flow rate of 2 gallons per minute (7.57 litres per minute) to a 175 gallon (662 litre) continuous stirred tank reactor containing internal baffles and a 1/2 horsepower (373 W) agitator. 5.0 molar sulfuric acid solution was fed to the reactor at a flow rate of 15 milliliters per minute to maintain the desired pH of 2.10 in the reactor. The reactor was operated at an average temperature of 115°F (46°C) with a liquid residence time of 87.5 minutes. 93.8% of the ethylene oxide in solution was converted to ethylene glycol. All ethylene oxide and ethylene glycol determinations were made by gas chromatography with simultaneous measure-ments at the inlet and oulet of the reactor.

## Claims

1. A process for removing at substantially ambient pressure an olefin oxide having 2 or 3 carbon atoms from a gas composition containing said olefin oxide and converting the olefin oxide to products including alkylene glycol which process comprises:

contacting said gas composition with an adsorbent capable of adsorbing an olefin oxide;

regenerating the olefin oxide from the adsorbent periodically to provide a more concentrated olefin oxide gas composition;

contacting said concentrated olefin oxide gas composition with an aqueous absorbing liquid at substantially ambient pressure and an initial temperature less than about 80°F (27°C), causing absorption of at least 97% of the olefin oxide into the aqueous liquid;

feeding the aqueous liquid containing the absorbed olefin oxide at a temperature of less than about 80°F (27°C) and an acid catalyst into a reactor, said reactor being at atmospheric pressure or slightly above atmospheric pressure;

reacting said aqueous liquid for a time sufficient to convert at least97% of the olefin oxides to products including an alkylene glycol while continuously removing sufficient quantities of aqueous liquid from the reactor to keep the volume of solution in the reactor substantially constant; and

recycling the aqueous liquid leaving the reactor, back to the absorber for further absorption of olefin oxide.

2. The process according to claim 1 wherein the adsorbent comprises a bed of activated carbon.

3. The process as claimed in any preceding claim wherein the volume of olefin oxide in the gas composition is between 0 and 50%.

4. The process as claimed in any preceding claim wherein the aqueous liquid leaving the reactor is cooled and recycled back to the absorber for further absorption of olefin oxide.

5. The process as claimed in any preceding claim wherein the aqueous liquid is neutralized with an alkali on removal from the reactor.

6. The process as claimed in any preceding claim wherein the mole ratio of acid catalyst to olefin oxide is between about 0.01 and 25.

7. The process as claimed in any preceding claim wherein the pH in the reactor is between 0 and 2.

8. The process as claimed in any preceding claim wherein fresh water is introduced continuously into the absorber at a rate of between about 40 and 70 gal/1000 ft$^3$ (5.35 to 9.30 litres/m$^3$) gas.

9. The process as claimed in any preceding claim wherein the temperature in the reactor is less than about 80°F (27°C).

10. The process as claimed in any preceding claim wherein the acid catalyst is sulfuric acid.

11. The process as claimed in any preceding claim wherein the hydrogen ion concentration of the acid catalyst in the reactor is about 0.01 to 1.0 gram-mole per litre.

12. The process as claimed in any preceding claim wherein the initial pH of the absorbing liquid is between 0.2 and 8.

## Patentansprüche

1. Verfahren zum Entfernen eines Olefinoxids mit zwei oder drei Kohlenstoffatomen bei im wesentlichen Umgebungsdruck aus einem Gasgemisch, das das Olefinoxid enthält und zum Umwandeln des Olefinoxids in Produkte, die Alkylenglykol enthalten mit folgenden Verfahrensschritten:

— Inkontaktbringen des Gasgemisches mit einem Adsorbens, das in der Lage ist, Olefinoxid zu adsorbieren;

— periodische Rückgewinnung des Olefinsoxids aus dem Adsorbens, um ein Olefinoxid-Gasgemisch mit höherer Konzentration zu erhalten;

— Inkontaktbringen des konzentrierten Olefinoxid-Gasgemisches mit einer wässrigen, absorbierenden Flüssigkeit im wesentlichen bei Umgebungsdruck und einer Anfangstemperatur, die unterhalb 80°F (27°C) liegt, wodurch eine Absorption von zumindest 97% des Olefinoxids in der wässrigen Flüssigkeit erzielbar ist;

enthaltenden wässrigen Flüssigkeit bei einer Temperatur von weniger als 80°F (27°C) und eines sauren Katalysators in einen Reaktor, wobei im Reaktor Atmosphärendruck oder ein geringfügig höherer als Atmosphärendruck herrscht;

— Reaktion der wässrigen Flüssigkeit während einer Zeitspanne, die ausreicht, um zumindest 97% des Olefinoxids in Produkte umzuwandeln, die Alkylenglykol enthalten, während kontinuierlich genügen de Mengen von wässriger Flüssigkeit aus dem Reaktor entfernt werden, um das Volumen der Lösung im Reaktor im wesentlichen konstant zu halten; und
— Rückführung der den Reaktor verlassenden wässrigen Flüssigkeit zum Absorber zur weiteren Absorbierung von Olefinoxid.

2. Verfahren nach Anspruch 1, wobei das Adsorbens eine Schicht von Aktivkohle enthält.

3. Verfahren nach einem der vorgenannten Ansprüche, wobei der Volumenanteil des Olefinoxids im Gasgemisch zwischen 0 und 50% liegt.

4. Verfahren nach einem der vorgenannten Ansprüche, wobei die den Reaktor verlassende wässrige Flüssigkeit gekühlt und zum Absorber zur weiteren Absorbierung von Olefinoxid zurückgeführt wird.

5. Verfahren nach einem der vorgenannten Ansprüche, wobei die wässrige Flüssigkeit nach dem Verlassen des Reaktors mit einem Alkali neutralisiert wird.

6. Verfahren nach einem der vorgenannten Ansprüche, wobei das Mol-Verhältnis des saueren Katalysators zum Olefinoxid zwischen, 0,01 und 25 liegt.

7. Verfahren nach einem der vorgenannten Ansprüche, wobei der pH-Wert im Reaktor zwischen 0 und 2 liegt.

8. Verfahren nach einem der vorgenannten Ansprüche, wobei in den Absorber kontinuierlich Frischwasser mit einer Rate von ungefähr 40 bis 70 gal/1000 ft³ (5.35 bis 9.30 l/m³) Gas eingeführt wird.

9. Verfahren nach einem der vorgenannten Ansprüche, wobei die Temperatur im Reaktor niedriger als ungefähr 80°F (27°C) liegt.

10. Verfahren nach einem der vorgenannten Ansprüche, wobei der sauere Katalysator Schwefelsäure ist.

11. Verfahren nach einem der vorgenannten Ansprüche, wobei die Wasserstoff-Ionen-Konzentration die saueren Katalysators im Reaktor ungefähr bei 0.01 bis 1.0 Gramm-Mol pro Liter liegt.

12. Verfahren nach einem der vorgenannten Ansprüche, wobei der Anfangs-pH-Wert der absorbierenden Flüssigkeit zwischen 0.2 und 8 liegt.

## Revendications

1. Procédé permettant d'extraire, sensiblement à la pression ambiante, un oxyde d'oléfine présentant 2 ou 3 atomes de carbone à partir d'une composition gazeuse contenant un tel oxyde d'oléfine, et de convertir cet oxyde d'oléfine en produits comprenant un alcoylène-glycol, ce précédé consistant:
à mettre ladite composition gazeuse au contact d'un adsorbant capable d'adsorber un oxyde d'oléfine,
à régénérer périodiquement l'oxyde d'oléfine à partir de cet adsorbant de façon à fournir une composition gazeuse plus concentrée en oxyde d'oléfine,
à mettre cette composition gazeuse concentrée en oxyde d'oléfine au contact d'un liquide absorbant aqueux sensiblement à la pression ambiante et à une température initiale inférieure à environ 80°F (27°C), afin de provoquer l'absorption d'au moins 97% de l'oxyde d'oléfine dans ce liquide aqueux,
à amener dans une enceinte de réaction d'une part le liquide aqueux contenant l'oxyde d'oléfine absorbé et se trouvant à une température inférieure à environ 80°F (27°C) et d'autre part un catalyseur acide, cette enceinte de réaction se trouvant à la pression atmosphérique ou légèrement au-dessus de celle-ci,
à faire réagir ledit liquide aqueux pendant une période de temps suffisante pour convertir au moins 97% d'oxyde d'oléfine en produits comprenant un alcoylène-glycol, tout en extrayant en continu du réacteur des quantités de liquide aqueux suffisantes pour maintenir sensiblement constant le volume de solution présent dans cette enceinte de réaction et
à recycler vers l'enceinte d'absorption le liquid aqueux quittant cette enceinte de réaction, en vue d'une nouvelle absorption de l'oxyde d'oléfine.

2. Procédé selon la revendication 1, dans lequel l'adsorbant est constitué par un lit de charbon actif.

3. Procédé tel que revendiqué dans une revendication précédente quelconque, dans lequel le volume d'oxyde d'oléfine se trouvant dans la composition gazeuse est compris entre 0 et 50%.

4. Procédé tel que revendique dans une revendication précédente quelconque, dans lequel on refroidit le liquide aqueux quittant l'enceinte de réaction et on le recycle vers l'enceinte d'absorption en vue d'une nouvelle absorption de l'oxyde d'oléfine.

5. Procédé tel que revendiqué dans une revendication précédente quelconque, dans lequel on neutralise le liquide aqueux à l'aide d'une base lors de son extraction hors de l'enceinte de réaction.

6. Procédé tel que revendiqué dans une revendication précédente quelconque, dans lequel le rapport molaire du catalyseur acide à l'oxyde d'oléfine est compris entre 0,01 et 25.

7. Procédé tel que revendiqué dans une revendication précédente quelconque, dans lequel le pH régnant dans l'enceinte de réaction est compris entre 0 et 2.

8. Procédé tel que revendiqué dans une revendication précédente quelconque, dans lequel on introduit en continue de l'eau fraîche dans l'enceinte d'absorption sous en débit compris entre environ 40 et 70 gallons/1000 pieds³ de gaz (5,35 à 9,30 litres/m³).

9. Procédé tel que revendique dans une revendication précédente quelconque, dans lequel la température régnant dans l'enceinte de réaction est inférieure à environ 80°F (27°C).

10. Procédé tel que revendiqué dans une revendication précédente quelconque, dans lequel le catalyseur acide est de l'acide sulfurique.

11. Procédé tel que revendiqué dans une revendication précédente quelconque, dans lequel la concentration en ions hydrogène du catalyseur acide se trouvant dans l'enceinte de réaction est d'environ 0,01 à 1,0 gramme-mole par litre.

12. Procédé tel que revendiqué dans une revendication précédente quelconque, dans lequel le pH initial du liquide absorbant est compris entre 0,2 et 8.

OLEFIN OXIDE-CONTAMINATED GAS STREAM — 10

COOLING WATER — 24

PUMP 26 — 14

12 — 18 — 20

HEAT EXCHANGER 22 — 28 — 34

COOLING WATER TO COOLING TOWER — 32

CONDENSATE — 30

ABSORBING LIQUID — 36

PUMP 38 — 40

ABSORBER 16 — 44

ABSORBER OUTLET LIQUID — 42

72

OLEFIN OXIDE-FREE GAS STREAM

CHILLED WATER — 74

HEAT EXCHANGER 70

CHILLED WATER TO CHILLER SYSTEM — 76

ACID — 46

PUMP 48 — 50

PUMP 52 — 54

REACTOR 56 — 58

68

NEUTRALIZING LIQUID — 60

PUMP 62 — 64

TO WATER TREATMENT SYSTEM — 66

1